# EUROPÄISCHE PATENTSCHRIFT

(11) **EP 2 283 894 B1**
(45) Veröffentlichungstag und Bekanntmachung des Hinweises auf die Patenterteilung: **02.09.2015**
(21) Anmeldenummer: 10155869.0
(22) Anmeldetag: 09.03.2010
(51) Int. Cl.: A61N 1/05, A61N 1/362, A61N 1/39, A61N 1/36, A61N 1/08

(54) **Stimulationselektrodenleitung**
Stimulation electrode lead
Conduite d'électrodes de stimulation

(30) Priorität: 02.04.2009 DE 102009002152
(43) Veröffentlichungstag der Anmeldung: 16.02.2011
(73) Patentinhaber: Biotronik CRM Patent AG, 6341 Baar (CH)
(72) Erfinder: Weiss, Ingo, 12435 Berlin (DE); Knorr, Stefan, 10119 Berlin (DE); Maxfield, Michelle, 10967 Berlin (DE); Friedrich, Michael, 14532 Kleinmachnow (DE)
(74) Vertreter: Galander, Marcus

(56) Entgegenhaltungen:
- WO-A1-02/083016
- US-A- 5 545 201
- US-A1- 2007 299 493
- US-A1- 2008 071 313

## Beschreibung

Die Erfindung betrifft eine implantierbare Stimulationselektrodenleitung für medizinische Anwendungen zur elektrischen Stimulation eines biologischen Gewebes und/oder Organs und eine implantierbare Stimulationsanordnung.

Langgestreckte Elektrodenleitungen, insbesondere Stimulationselektrodenleitungen, stellen eine wichtige Baugruppe vieler elektrischer Systeme dar, die zu diagnostischen und/oder therapeutischen Zwecken in der Medizin eingesetzt werden. Dies können z. B. Herzschrittmacher, Defibrillatoren, Neurostimulatoren oder Kathetersysteme sein.

Fig. 1 zeigt den Grundaufbau einer implantierbaren Defibrillationsanordnung 1, wie sie aus der US 5,531,766 bekannt ist. Um das Herz H eines Patienten P zu stimulieren, steht ein Defibrillator 3 in elektrischer Verbindung mit einer Elektrodenleitung 5', welche hier eine spezielle Elektrode ("Schockelektrode") 7 trägt, die sich an ihrem distalen Ende befindet und im Herzen des Patienten verlegt wird. Die Defibrillation erfolgt in der Regel über einen Strompfad zwischen dieser Elektrode und einer Gegenelektrode, die mehr oder weniger entfernt von dem zu stimulierenden Herzen liegt. Das Gehäuse 8 des Defibrillators 3, das die Einheiten zur Detektion von Herzsignalen und Erzeugung von elektrischen Impulsen enthält, kann als Gegenelektrode wirken. Alternativ kann eine Gegenelektrode an der Elektrodenleitung oder am Gehäuse außen angebracht sein.

Funktionswesentlich bei Stimulationselektrodenleitungen ist eine Zuleitungsstruktur, deren distales Ende leitfähige Kontakte oder Elektroden umfasst, die eine elektrische Verbindung zum umliegenden Gewebe und/oder Organ eines Patienten herstellen und die für Abtast- und/oder Stimulationszwecke vorgesehen sind. Besitzt eine Stimulationselektrodenleitung mehrere solcher Elektroden, so besteht die Zuleitungsstruktur auch aus mehreren voneinander isolierten Leitern, die häufig koaxial angeordnet sind.

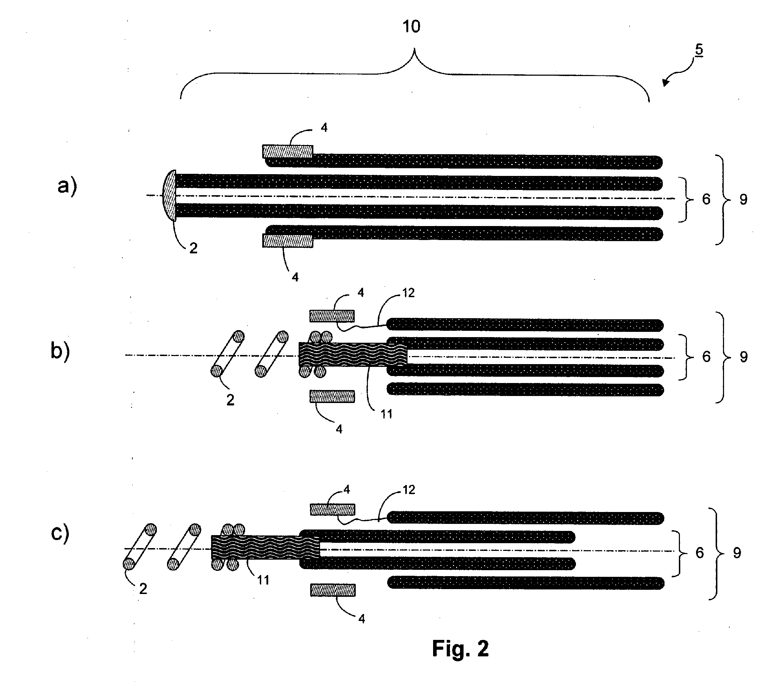

Figur 2 zeigt eine schematische Darstellung einer Standard-Stimulationselektrodenleitung 5, in der zwei Elektroden 2, 4 am distalen Ende der Zuleitungsstruktur 10 vorhanden sind, wobei eine Elektrode (Spitzenelektrode) 2 einen Kontakt an der Spitze der Leitungsstruktur 10 und die andere Elektrode (Ringelektrode) 4 einen Ringkontakt um die Leitungsstruktur 10 herum bildet. Da zwei Elektroden vorhanden sind, umfasst die Zuleitungsstruktur 10 zwei Leitungen 6, 9, die koaxial angeordnet sind.

Die Stimulationselektrodenleitungen können passiv fixierbare Elektrodenleitungen (Fig. 2a), wobei die Innenzuleitung 6 relativ zur Außenzuleitung 9 fest ist, oder aktiv fixierbare Elektrodenleitungen (Fig. 2b-c) sein, wobei die Innenzuleitung 6 relativ zur Außenzuleitung 9 beweglich ist. Im letzteren Fall, verschiebt sich die Innenzuleitung 6 relativ zu der Außenzuleitung 9 durch Schrauben, um die Spitzenelektrode 2 ins Gewebe einzuführen bzw. zu schrauben. Insbesondere hat hierbei eine spezielle Spitzenelektrode 2 die Form einer Schraubwendel.

In beiden Konfigurationen führt die äußere Zuleitung 9 der Zuleitungsstruktur 10 zur Ringelektrode 4 und die innere Zuleitung 6 zur Spitzenelektrode 2. Der Kontakt zwischen den Zuleitungen und den Elektroden erfolgt entweder direkt (Fig. 2a) oder durch ein leitfähiges Verbindungselement (Fig. 2b-c), welches ein passender Montagestift 11 oder ein elektrisches Kabel 12 ist.

Obwohl diese Bauweise aus mechanischer Sicht naheliegend und konstruktiv einfach umsetzbar ist, kann sie aber aus elektrischer Sicht negative Effekte haben. Es ist bekannt, dass solche Stimulationselektrodenleitungen, wenn sie einem von modernen Untersuchungs- und Diagnostikgeräten erzeugten elektromagnetischen Feld ausgesetzt werden, zu bestimmten Störungsproblemen führen.

Es ist daher die Aufgabe der Erfindung, eine verbesserte Stimulationselektrodenleitung bzw. Stimulationsanordnung anzugeben, die solche Probleme stark reduziert oder beseitigt. Eine implantierbare Stimulationselektrodenleitung umfasst, gemäß der vorliegenden Erfindung, eine koaxiale Leitungsstruktur mit einer Innenzuleitung und einer Außenzuleitung, wobei die zwei Zuleitungen voneinander elektrisch isoliert sind. Am oder nahe dem distalen Ende der Leitungsstruktur sind eine erste und eine zweite Elektrode vorgesehen, wobei die erste Elektrode an der Spitze der Leitungsstruktur und die zweite Elektrode proximal von der ersten Elektrode angeordnet ist.

Die erfindungsgemäße Stimulationselektrodenleitung zeichnet sich dadurch aus, dass die elektrischen Verbindungen zwischen den Zuleitungen und den Elektroden derart "überkreuzt" sind, dass die Innenzuleitung zur zweiten Elektrode und die Außenzuleitung zur ersten Elektrode geführt sind.

Diese Lösung kann sowohl für eine passiv fixierbare Stimulationselektrodenleitung, wobei die Innenzuleitung relativ zur Außenzuleitung fest ist, als auch für eine aktiv fixierbare Stimulationselektrodenleitung, wobei die Innenzuleitung relativ zur Außenzuleitung beweglich ist, angewendet werden. Im letzteren Fall kann die Innenzuleitung gegenüber der Außenzuleitung einfach verschiebbar oder drehbar bzw. schraubbar sein, wobei die erste Elektrode etwa eine Spitze mit Widerhaken oder eine Schraube sein kann, der/die mit der Innenzuleitung ins Gewebe eingeführt bzw. geschraubt wird. Im Besonderen kann die erste Elektrode einen Kontakt der kompletten Spitze der Leitungsstruktur und die zweite Elektrode einen Ringkontakt um die Leitungsstruktur herum bilden. Außerdem kann die Elektrodenleitung mono- oder bipolar sein.

Bei der aktiv fixierbaren Stimulationselektrode sind verschiedene mechanische Konfigurationen möglich. In einer ersten Konfiguration wird die Innenzuleitung durch Drehen bewegt und ihr Ende gegenüber dem Ende der Außenzuleitung verschoben. Da beide Zuleitungen am proximalen Ende in fester Lagebeziehung zueinander sind, muss sich hierbei die Außenzuleitung stauchen und/oder die Innenzuleitung dehnen. Die Drehung und die Verschiebung der Innenzuleitung werden hierbei durch ein Übertragungselement auf die erste Elektrode übertragen. Das Übertragungselement, das eine geringe Längssteifigkeit besitzt, verbindet die Innenzuleitung mit der ersten Elektrode, ohne einen galvanischen Kontakt herzustellen, womit nur mechanische Lasten (Kräfte und Momente) der Innenzuleitung übertragen werden.

In einer zweiten Konfiguration wird die Innenzuleitung nur gedreht und verschiebt sich nicht gegenüber der Außenzuleitung. Daraus folgt, dass die Zuleitungen durch das Drehen weder gedehnt noch gestaucht und der Kraftaufwand zum Ausdrehen der ersten Elektrode verringert und die mechanische Beanspruchung der Zuleitungen reduziert werden. Das Moment zum Ausdrehen der ersten Elektrode wird hierbei von der Innenzuleitung ebenfalls über ein Übertragungselement übertragen. Dieses Element kann seine Länge verändern, um die erste Elektrode relativ zur zweiten Elektrode zu verschieben.

Um die erste Elektrode ins Gewebe einzuführen und dort zu fixieren, kann ein Mittel zur aktiven Fixierung mit dem Übertragungselement wirken. Dieses Mittel kann zum Beispiel eine Feder sein, die durch einen Druck oder ein Moment auf das Übertragungselement wirkt, um die erste Elektrode in einer bestimmten Position zu halten.

Die Verbindung zwischen den Elektroden und den Zuleitungen kann durch elektrische Verbindungsleitungen, Drähte oder Kabel erfolgen. Eine Verdrehung der Innenzuleitung gegenüber der Außenzuleitung hat jedoch auch eine Verdrehung und Dehnung der verbindenden Drähte zur Folge. Daher können flexible elektrische Drähte als Verbindungsleitungen angewendet werden. Vorzugsweise können diese flexible Drähte aus dehnbarem Material bestehen, um ein unerwünschtes Verflechten der Drähte beim Ein- bzw. Ausschrauben der Innenzuleitung zu vermeiden.

In einer Ausführungsform der Erfindung werden die sich kreuzenden elektrischen Verbindungsleitungen als Wendelstruktur ausgeführt. Diese Wendelstruktur kann längs des zwischen der Innenzuleitung und der ersten Elektrode angeordneten Übertragungselements oder um dieses herum ausgebildet werden. Beim Ausdrehen der Innenzuleitung bzw. der Spitzenelektrode wird die Wendelstruktur in Längsrichtung verlängert und die Anzahl der Windungen reduziert. Damit wird beim Drehen die mechanische Belastung auf die elektrischen Verbindungsleitungen stark verrindert. In einem bevorzugten Aufbau wird die elektrische Verbindung von der Innenzuleitung bis nach vorne, längs des Übertragungselements, durchgeführt und anschließend als Wendel bis zur zweiten Elektrode zurückgeführt. Die elektrische Verbindung der Außenzuleitung wird dagegen direkt als Wendel an die erste Elektrode geführt.

Als Material für die elektrischen Verbindungen werden flexible Drähte, Litzen, Seile oder ähnliches verwendet. Mindestens eine der Verbindungsleitungen muss isoliert sein, um einen Kurzschluss zu verhindern. Zweckmäßigerweise sind beide flexible Verbindungsleitungen voneinander isoliert. Um den Durchmesser der Wendelstruktur beim Ausdrehen nicht zu verringern, besitzt die Wicklung der elektrischen Drähte vorzugsweise eine Gegendrehrichtung zur Ausdrehrichtung.

Alternativ zu einem isolierenden Übertragungselement zwischen der Innenzuleitung und der ersten Elektrode kann eine leitfähige Struktur verwendet werden, die nur am distalen Ende isoliert ist. Dadurch kann eine elektrische Leitung, welche die Innenzuleitung mit dem distalen Ende der Wendelstruktur verbindet, entfallen.

Jedoch können die Verbindungsleitungen um die Innenzuleitung herum als Wendelstruktur geführt werden. Im Gegensatz zu den obigen Lösungen werden in einer solchen Konstruktion die mechanischen Eigenschaften der Stimulationselektrodenleitung nur wenig beeinflusst und führen kaum zu einer Versteifung im distalen Bereich der Stimulationselektrodenleitung.

Wenn die Geometrieparameter der Wendelstruktur richtig dimensioniert sind, verändert sich der Durchmesser der Wendel beim Ausdrehen der Spitzenelektrode nicht, so dass sehr wenig Bauraum in der Elektrodenleitung benötigt wird. Eine solche isodiametrische Wendelstruktur hat den Vorteil, dass die gesamte Wendelverformung in einem Zylinderring ablaufen kann. Die Dicke des Zylinderrings ist hierbei nur unwesentlich größer als der Drahtdurchmesser, somit wird eine Überkreuzung benachbarter elektrischer Verbindungsleitungen vermieden.

Zweckmäßigerweise kann die Wendel auch aus koaxialen Verbindungsleitungen, mehradrigen Litzen, Drähten mit eckigem Querschnitt oder anderen Leitungsstrukturen bestehen. In einer anderen Ausführungsform hat das Übertragungselement die Form einer Hohlspirale. Im Inneren der Spirale können die Drähte verlaufen, die die Innenzuleitung mit der zweiten Elektrode und die Außenzuleitung mit der ersten Elektrode verbinden. Die Spirale kann komplett aus isolierendem Material bestehen, oder bis auf den vorderen Teil, der in Kontakt mit der ersten Elektrode steht, leitfähig sein. Beim Ausdrehen der Innenzuleitung verdrehen sich die Drähte innerhalb der Spirale. Falls die erste Elektrode die Form einer Schraube hat, kann vorzugsweise die Steigung der Spirale der Schraubensteigung gleichen. Somit ist die Stelle, an der die Drähte in die Spirale reichen, von der Lage der Schraube unabhängig.

In einer alternativen Ausführung der Erfindung kann eine Spiralfeder anstelle von flexiblen Verbindungsleitungen zur elektrischen Verbindung der Innenzuleitung mit der zweiten Elektrode und der Außenzuleitung mit der ersten Elektrode verwendet werden. Die Spiralfeder kann einen äußeren und einen inneren Teil haben, wobei der äußere Teil bewegungslos ist und der innere Teil sich gegenüber den äußeren Teil verdrehen kann. Im Besonderen verformt sich die Feder beim Ausdrehen der Innenzuleitung bzw. der ersten Elektrode. Die Spiralfeder kann aus Metall, isoliertem Metall, metallisiertem Kunststoff oder anderen leitfähigen Strukturen bestehen.

Wenn mehr als eine Leitung notwendig ist, kann für jede Leitung eine eigene Spiralfeder oder alternativ eine einzige Feder mit mehreren Leitungen genutzt werden.

Die Spiralfeder kann aus einem gewickelten Substrat bestehen, auf dem sich beidseitig eine Metallisierung befindet, wobei die Metallisierung auf einer Seite des Substrats die Verbindung der Innenzuleitung mit der zweiten Elektrode, und die Metallisierung auf der anderen Seite des Substrats die Verbindung der Außenzuleitung mit der ersten Elektrode, herstellt. In einem möglichen Aufbau ist eine der Metallisierungsschichten vollständig isoliert.

In einer weiteren zweckmäßigen Ausführung der Erfindung wirkt die Federkraft der Spiralfeder jeder Drehung der Innenzuleitung bzw. der ersten Elektrode entgegen. Aus diesem Grund sollte die Federsteifigkeit möglichst gering sein, um eine Drehung ohne großen Widerstand zu ermöglichen. Eine geringe Steifigkeit kann durch eine hohe Anzahl von Windungen und eine geringe Biegesteifigkeit erreicht werden. Die Ruhelage der Feder stellt hierbei die ausgeschraubte Lage dar, um zu vermeiden, dass die Rückstellkraft der Feder die erste Elektrode unbeabsichtigt wieder zurückschraubt. Hierbei wird eine Stimulationselektrodenleitung mit einer zurückgezogenen ersten Elektrode hergestellt, damit die Feder vorgespannt ist. Beim Ausdrehen der Innenzuleitung bzw. der ersten Elektrode entspannt sich die Feder, wodurch die Ausschraubbewegung unterstützt wird. In dieser Konfiguration führt die Feder zur aktiven Fixierung der ersten Elektrode.

Anstelle einer vollständigen Metallisierung des Substrats kann eine der beiden Leitungen am Ende des Substrats durchkontaktiert werden. Dies hat den Vorteil, dass die Kontaktierung der metallisierten Flächen von nur einer Seite, der inneren oder der äußeren, möglich ist.

Als weitere Ausführungsform können die Enden des Substrats derart gebogen sein, dass eine Spiralfeder mit einem variablen Durchmesser hergestellt wird, so dass Teile der Elektrodenleitung mit verschiedenen Durchmessern miteinander verbunden werden können.

In einer anderen Ausführung der Erfindung kann ein leitfähiger Adapter zwischen der Spiralfeder und der ersten bzw. der zweiten Elektrode und/oder der Innen- bzw. der Außenzuleitung vorhanden sein, der andere mechanische Eigenschaften als die des Substrats der Spiralfeder aufweist. Somit kann der direkte Kontakt zwischen dem Substrat und den Komponenten der Elektrodenleitung vermieden werden, um eine verbesserte mechanische Stabilität zu erreichen. Dieser Adapter kann ein Keramikröhrchen sein, das auf seiner Oberfläche mehrere Metallisierungen besitzt, welche die elektrische Verbindung zwischen den Metallisierungsschichten der Feder und den Elektroden bzw. den Zuleitungen gewährleisten. Hierbei kann der Kontakt der zweiten Elektrode durch die äußere Metallisierung des Adapters ersetzt werden.

In einer weiteren Ausführung der Erfindung kann eine röhrenförmige Struktur mit integrierter Leitungsüberkreuzung anstelle von flexiblen Verbindungsleitungen oder der Spiralfeder zur elektrischen Verbindung der Innenzuleitung mit der zweiten Elektrode und feder zur elektrischen Verbindung der Innenzuleitung mit der zweiten Elektrode und der Außenzuleitung mit der ersten Elektrode verwendet werden. Basis dieser Struktur kann ein Keramikröhrchen sein, dessen Innen- und Außenseite derartig metallisiert ist, dass mehrere Durchkontaktierungen zur gewünschten Überkreuzung führt. Dabei können die äußere Form der Struktur sowie die Kontaktflächen zylindrisch sein.

Um die Flexibilität der Innenzuleitung relativ zur Außenzuleitung zu erhöhen, kann die röhrenförmige Struktur mit einer distalen leitfähigen Wendel zur Verbindung der Außenzuleitung mit der ersten Elektrode und mit einer proximalen leitfähigen Spirale, parallel zur distalen Spirale, zur Verbindung der Innenzuleitung mit der zweiten Elektrode gekoppelt werden. Anders als die oben erwähnte Spiralfeder bestehen diese distalen und proximalen Wendeln nicht aus einem gewickelten metallisierten Substrat. Das Substrat ist hierbei nicht erforderlich, und die Spirale kann gegebenenfalls aus isoliertem Metallband bestehen, welches zu einer Wendel aufgewickelt wird. Als Vorteil benötigt diese Spirale weniger Bauhöhe, und die geringere Steifigkeit dieser Spiralen erzeugt ein geringeres unerwünschtes Rückstelldrehmoment.

In obiger Ausführungsform mit zwei parallel angeordneten Spiralen besteht jedoch die Schwierigkeit, darin die Leitungsüberkreuzung zu erhalten. Um die Kreuzung zu umgehen, kann vorzugsweise die Verbindungsleitung von der Außenzuleitung zur ersten Elektrode außen über die zweite Elektrode geführt werden. In alternativer Weise kann die zweite Elektrode, die einen Ringkontakt bildet, z. B. eine Bohrung aufweisen, durch die die Leitung geführt wird, oder der Ringkontakt kann durch ein Zylinderringsegment ersetzt werden, durch dessen Spalt eine Leitung geführt wird oder der Ringkontakt kann aus einem Isolator bestehen, der teilmetallisiert ist und eine leitfähige Durchführung besitzt.

Zur Übertragung der elektrischen Signale zwischen zwei gegeneinander verdrehten Zuleitungen können statt der Spiralfedern bzw. Wendeln auch Schleifkontakte verwendet werden. Denkbar ist auch eine Konstruktion ähnlich der eines Kugellagers, wobei die Kugeln, deren Oberfläche leitfähig ist, zur Übertragung der elektrischen Signale genutzt werden. Alternativ kann auch leitfähige Flüssigkeit eingesetzt werden, die sich in einem dünnen Spalt zwischen zwei leitfähigen Kontakten befindet und diese miteinander elektrisch verbindet. Wenn der Spalt dünn genug ist, können auch Substanzen mit einem relativ hohen Widerstand genutzt werden. Die Nutzung von flüssigen Metallen mit einer entsprechend hohen Leitfähigkeit ist ebenfalls denkbar. Das Prinzip der Kugellager kann mit demjenigen der leitfähigen Flüssigkeit beliebig kombiniert werden.

In den obigen Ausführungsformen besitzen alle Bauteile, die sich beim Ausdrehen aneinander vorbeibewegen, eine möglichst reibungsarme Oberfläche, um wenig mechanischen Widerstand zu erzeugen. Dies kann durch geeignete Beschichtungen, wie z. B. Teflon oder SilGlide, erreicht werden. Außerdem können die Wendelstrukturen der Verbindungsleitungen ggf. mit einem Schlauch überzogen, oder eingegossen werden, um eine glatte Oberfläche zu erzeugen. So können sich die Leitungen der Wendel beim Ausdrehen kaum verhaken.

In einer weiteren Ausführung der Erfindung kann die elektrische Kreuzung auch mehrfach in einer der Elektroden stattfinden. Sinnvoll kann eine weitere Kreuzung sein, um die gleiche Steckerbelegung (z. B. IS-1) wie bisherige Systeme zu erreichen. Dazu kann eine weitere Kreuzung direkt in den IS-1 Stecker integriert werden.

Das Konzept der gekreuzten Leitungen kann prinzipiell auch auf Elektrodenleitungen mit mehr als nur zwei Zuleitungen übertragen werden.

Eine erfindungsgemäße implantierbare Stimulationsanordnung zeichnet sich dadurch aus, dass eine der oben beschriebenen Stimulationselektrodenleitungen am proximalen Ende in Verbindung mit einem implantierbaren Gerät, z. B. einem Defibrillator, einem Herzschrittmacher oder einem Neurostimulator, steht. Das Gerät weist ein Gehäuse auf, welches in der Regel eine Steuereinheit zum Abfühlen bzw. zur Erzeugung elektrischen Impulse und eine Batterie zur Energieversorgung enthält. Zusätzlich zu Elektroden zur elektrischen Stimulation kann die Stimulationselektrodenleitung einen Sensor bzw. eine Abfühlelektrode z. B. zum Abfühlen von Gewebe- und/oder Organaktionspotentialen aufweisen.

Im Hinblick auf die seit langem etablierten implantierbaren Stimulationsanordnungen ist es von Vorteil, dass mit der erfindungsgemäßen Anordnung die Störungsprobleme infolge der Verwendung moderner Untersuchungsgeräte, die elektromagnetischen Felder erzeugen, beseitigt oder wenigstens reduziert werden.

Vorteile und Zweckmäßigkeiten der Erfindung ergeben sich im Übrigen aus der nachfolgenden Beschreibung ausgewählter Ausführungsbeispiele anhand der Figuren. Von diesen zeigen:

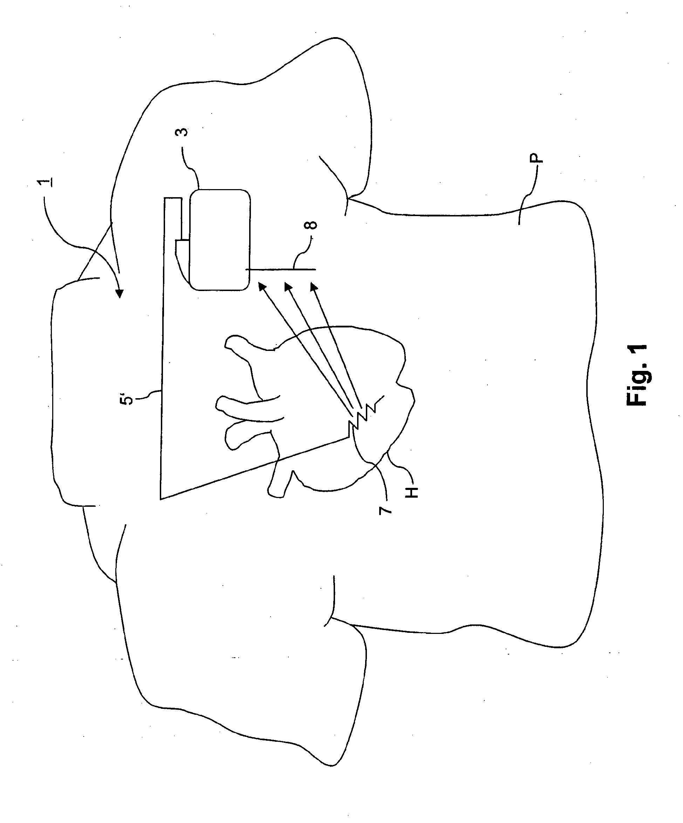

- Fig.1: eine schematische Darstellung einer implantierbaren Defibrillationsanordnung,
- Fig.2a bis 2c: schematische Darstellungen einer Stimulationselektrodenleitung gemäß dem Stand der Technik,
- Fig. 3a bis 3c: schematische Darstellungen einer Stimulationselektrodenleitung gemäß der Erfindung,
- Fig. 4a und 4b: schematische Darstellungen einer Stimulationselektrodenleitung mit einer Wendelstruktur zur elektrischen Verbindung gemäß einer Ausführungsform der Erfindung,
- Fig. 5a und 5b: schematische Darstellungen einer Stimulationselektrodenleitung mit einer Wendelstruktur zur elektrischen Verbindung gemäß alternativen Ausführungsformen der Erfindung,
- Fig. 6a und 6b: schematische Darstellungen einer Stimulationselektrodenleitung mit einer Hohlspirale als Übertragungselement gemäß einer Ausführungsform der Erfindung,
- Fig. 7a bis 7c: eine schematische Darstellung einer Spiralfeder zur elektrischen Verbindung gemäß der Erfindung,
- Fig. 8a bis 8d: schematische Darstellungen einer Stimulationselektrodenleitung mit einer Spiralfeder zur elektrischen Verbindung gemäß verschiedenen Ausführungsformen der Erfindung,
- Fig. 9: eine schematische Darstellung einer Stimulationselektrodenleitung mit einer röhrenförmigen Struktur mit integrierter Leitungsüberkreuzung zur elektrischen Verbindung gemäß einer Ausführungsform der Erfindung,
- Fig. 10a bis 10d: schematische Darstellungen abgewickelten Metallisierungsflächen einer röhrenförmigen Struktur mit integrierter Leitungsüberkreuzung gemäß einer Ausführungsform der Erfindung, und
- Fig. 11: eine schematische Darstellung einer Stimulationselektrodenleitung mit einer röhrenförmigen Struktur mit integrierter Leitungsüberkreuzung zur elektrischen Verbindung gemäß einer alternativen Ausführungsform der Erfindung.

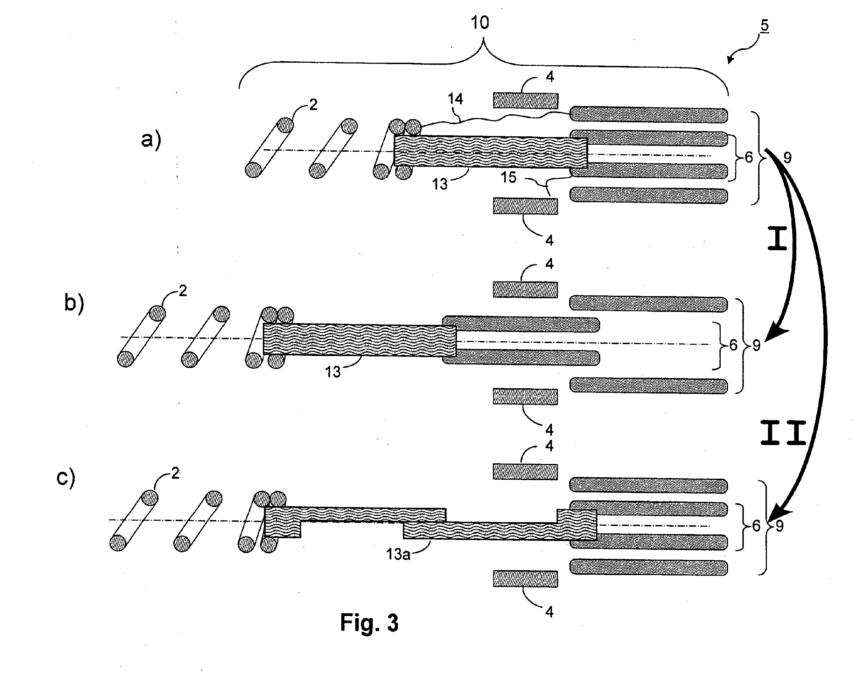

Fig. 3a zeigt das distale Ende einer implantierbaren Stimulationselektrodenleitung 5, insbesondere einer aktiv fixierbaren Stimulationselektrodenleitung 5, mit einer Leitungsstruktur 10, umfassend eine Innenzuleitung 6 und eine Außenzuleitung 9. Am distalen Ende der Leitungsstruktur 10 sind eine erste und eine zweite Elektrode 2, 4 vorhanden, wobei die erste Elektrode 2 die Form einer Schraubwendel (Schraubelektrode) und die zweite Elektrode 4 die Form eines Rings (Ringelektrode) um die Leitungsstruktur 10 herum hat. Die erste Elektrode 2 ist mit einem aus isoliertem Material bestehenden Übertragungselement 13 verbunden. Flexible und dehnbare elektrischen Leitungen 14, 15 stellen die elektrische Verbindung zwischen der Außenzuleitung 9 und der Schraubelektrode 2 bzw. der Innenzuleitung 6 und der Ringelektrode 4 her.

Fig. 3b zeigt eine Stimulationselektrodenleitung 5 im ausgeschraubten Zustand gemäß einer ersten Konfiguration (I), wobei die Innenzuleitung 6, relativ zur Außenzuleitung 9 durch Schrauben verschoben ist. Die Verdrehung der Innenzuleitung 6 gegenüber der Außenzuleitung 9 hat zur Folge, dass sich die verbindenden elektrischen Leitungen 14, 15 verdrehen und verlängern (in der Figur nicht dargestellt).

Fig. 3c zeigt eine Stimulationselektrodenleitung 5 im ausgeschraubten Zustand gemäß einer zweiten Konfiguration (II), wobei die Innenzuleitung 6 gegenüber der Außenzuleitung 9 nur gedreht wird und sich nicht verschiebt. Die Schraube 2 verschiebt sich relativ zur Ringelektrode 4 durch ein verlängerbares Übertragungselement 13b, welches beim Ausdrehen der Innenzuleitung 6 seine Länge verändert. Hierbei hat ebenfalls die Verdrehung der Innenzuleitung 6 gegenüber der Außenzuleitung 9 eine Verdrehung und Verlängerung der elektrischen Leitungen 14, 15 zur Folge (in der Figur nicht dargestellt).

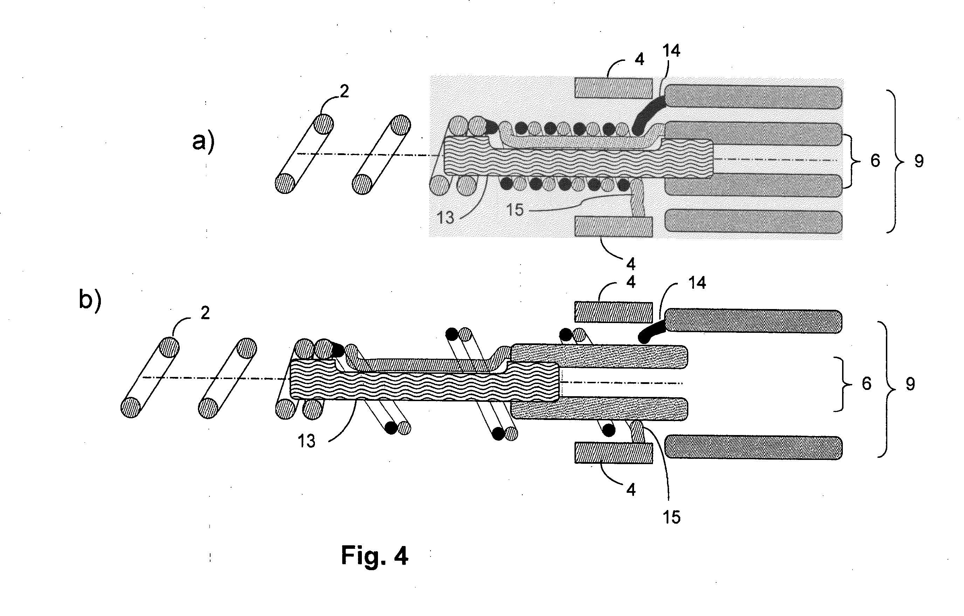

Fig. 4a zeigt das distale Ende einer Stimulationselektrodenleitung 5 wie in Figur 3a, in der beide elektrische Verbindungsleitungen 14, 15 als Wendel um das Übertragungselement 13 herum ausgeführt sind. Die elektrische Verbindung 15 ist von der Innenzuleitung 6 bis nach vorne, längs des Übertragungselements 13, durchgeführt und anschließend als Wendel bis zur Ringelektrode zurückgeführt. Die elektrische Verbindung 14 der Außenzuleitung 9 ist als Wendel direkt an die Schraubeelektrode geführt.

Fig. 4b zeigt den ausgeschraubten Zustand der Stimulationselektrodenleitung 5 der Fig. 4a. Beim Ausdrehen der Schraube 2 wird die Wendel verlängert und die Anzahl der Windungen reduziert.

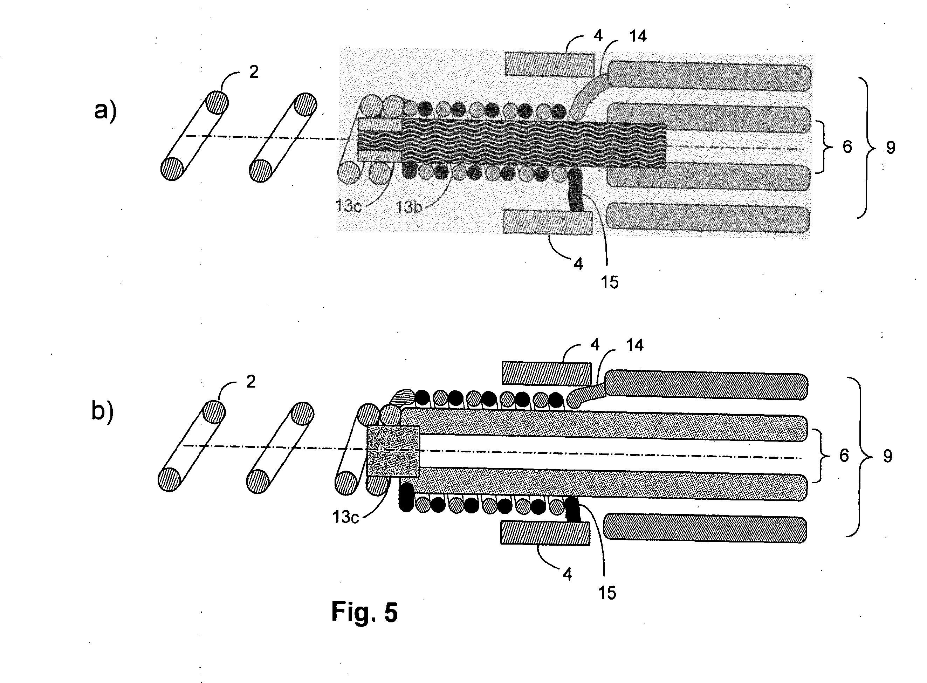

Fig. 5a zeigt eine Variante der Elektrodenleitung der Figur 4a, in der das Übertragungselement 13b aus einem leitfähigen Material besteht. Am distalen Ende des Übertragungselements 13b ist eine isolierende Struktur 13c vorhanden, um eine galvanische Verbindung zwischen der Innenzuleitung 6 und der Schraubeelektrode 2 zu verhindern. Wie aus der Figur zu entnehmen ist, ist die Durchführung der elektrischen Verbindung 15, von der Innenzuleitung 6 bis nach vorne längs des Übertragungselements 13b, nicht mehr nötig.

Fig. 5b zeigt eine weitere Variante der Elektrodenleitung der Figur 4A, in der die Wendelstruktur um die Innenzuleitung 6 herum ausgebildet ist. Um eine galvanische Verbindung zwischen der Innenzuleitung 6 und der Schraubeelektrode 2 zu verhindern, ist wiederum eine isolierende Struktur 13c zum Halten der Schraubeelektrode 2 vorhanden.

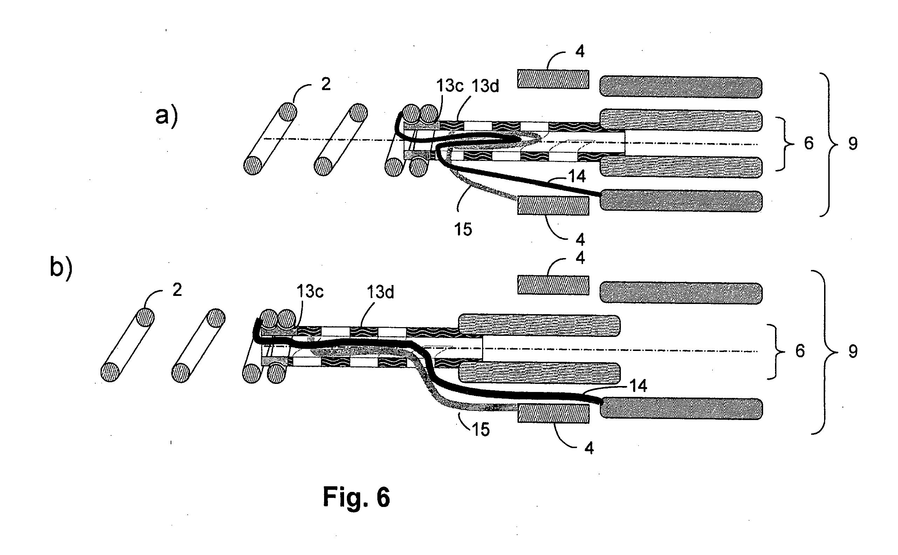

Fig. 6a und 6b zeigen das distale Ende einer Stimulationselektrodenleitung 5 ähnlich wie in Figur 3a, in der das Übertragungselement die Form einer Hohlwendel 13d hat, in der die Verbindungsleitungen 14, 15 verlaufen. Im Besonderen führen die Drähte 14, 15 im nicht ausgeschraubten Zustand (Fig. 6a) im distalen Bereich der Hohlwendel 13d hinein, während die Drähte 14, 15 im ausgeschraubten Zustand (Fig. 6b) im proximalen Bereich der Hohlspirale 13d hineinführen. Hierbei ist die Steigung der Schraubeelektrode 2 gleich der Steigung der Hohlwendel 13d, um die mechanische Belastung auf den Verbindungsleitungen 14, 15, beim Drehen, zu reduzieren. Die Hohlwendel 13d besteht hierbei aus leitfähigem Material, wobei der vordere Teil, der in Kontakt mit der ersten Elektrode 2 steht, elektrisch isoliert ist.

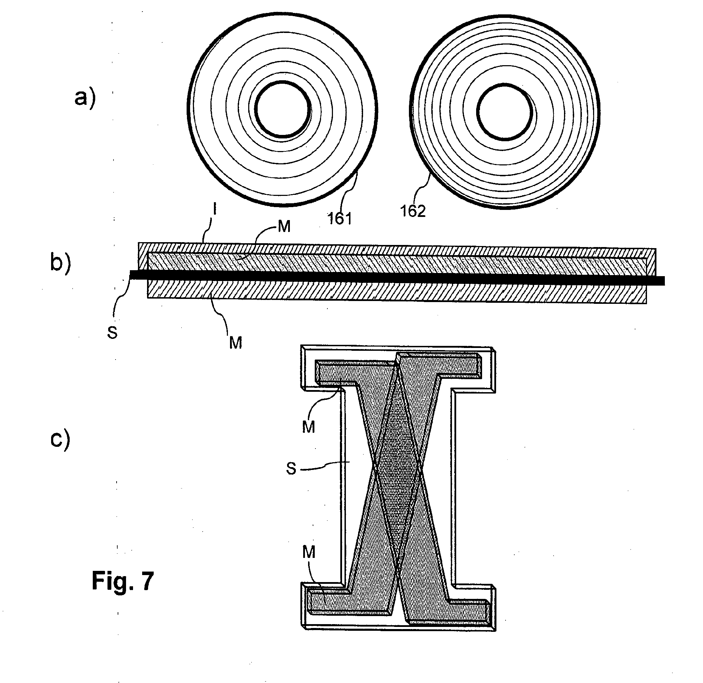

Fig. 7a bis 7c zeigen eine Ausführungsmöglichkeit einer Spiralfeder 16 zur elektrischen Verbindung für eine Stimulationselektrodenleitung 5. Die Spiralfeder umfasst einen inneren Teil 161 und einen äußeren Teil 162, welche die Kontakte zwischen den gegeneinander verdrehten Teilen der Elektrodenleitung 5 herstellen. Die Spiralfeder besteht aus vier Lagen: einem Substrat S, zwei Metallschichten M, die sich beidseitig auf dem Substrat S befinden, und einer Isolatorschicht I, die eine der Metallisierungsschichten vollständig bedeckt.

Wie in Fig. 7c gezeigt ist, ist eine Kunststofffolie S mit einer H-Form auf jeder Seite metallisiert, wobei jede Metallisierungsschicht M diagonal verläuft. Die Spiralfeder 16 wird hierbei bei der Aufwicklung der Kunststofffolie S erzeugt, wobei der mittlere Balken des H als eigentliche Feder 16 dient, während die Enden zur Kontaktierung der Metallisierungen M dienen.

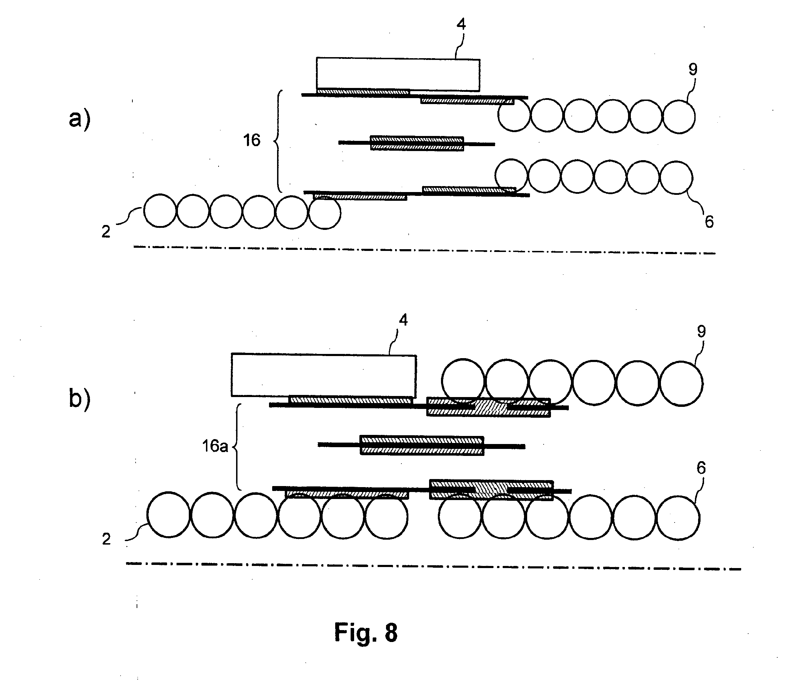

Fig. 8a zeigt den oberen Querschnitt des distalen Endes der Stimulationselektrodenleitung 5, in der die wie in Fig. 7 dargestellte Spiralfeder 16 zur elektrischen Verbindung zwischen der Innenzuleitung 6 und der zweiten Elektrode 4 bzw. zwischen der Außenzuleitung 9 und der ersten Elektrode 2 verwendet ist. Hierbei ist die H-Struktur der Fig. 7C in aufgerollter Form (zweifach gewickelt) und im Querschnitt an verschiedenen Stellen dargestellt. Die äußere Metallisierung der Spiralfeder 16 verbindet die zweite Elektrode 4 mit der Innenzuleitung 6, während die innere Metallisierung die erste Elektrode 2 mit der Außenzuleitung 9 kontaktiert.

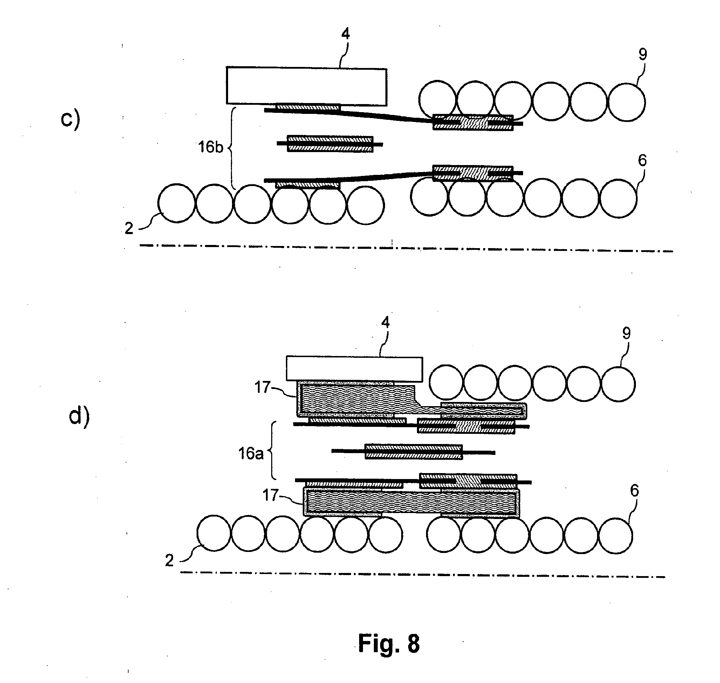

Fig. 8b zeigt eine Variante des Aufbaus der Spiralfeder, wobei zwei Durchkontaktierungen genutzt werden, um die Spiralfeder 16a nur von außen zu kontaktieren. Fig. 8c zeigt eine weitere Variante, wobei die Enden des Substrats S gebogen sind, um eine Spiralfeder 16b mit einem variablen Durchmesser zu erreichen.

Fig. 8d zeigt eine andere Ausführungsform, in der ein Keramikröhrchen 17 zwischen der Spiralfeder 16a, die nur Kontakte von außen hat, und den Komponenten der Elektrodenleitung 5 angeordnet ist. Das Keramikröhrchen 17 besitzt mehrere Metallisierungen, um die elektrische Verbindung zwischen der Innenzuleitung 6 und der zweiten Elektrode 4 bzw. zwischen der Außenzuleitung 9 und der ersten Elektrode 2, durch die Spiralfeder 16a, zu gewährleisten.

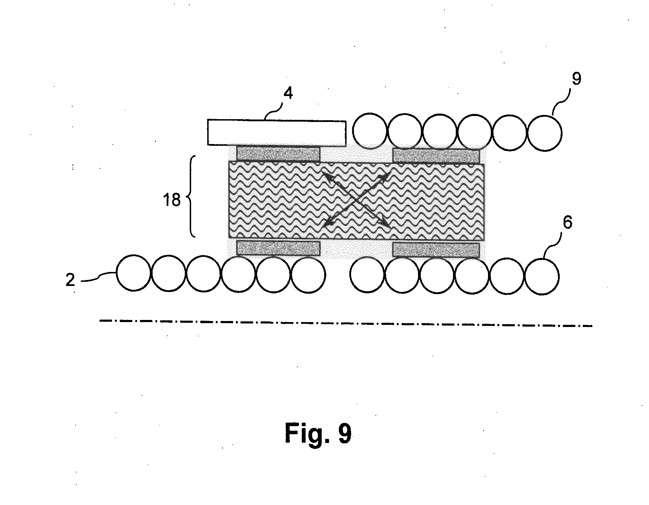

Fig. 9 zeigt den oberen Querschnitt des distalen Endes der Stimulationselektrodenleitung 5, in der ein Keramikröhrchen 18 mit integrierter Leitungsüberkreuzung zur elektrischen Verbindung zwischen der Innenzuleitung 6 und der zweiten Elektrode 4 bzw. zwischen der Außenzuleitung 9 und der ersten Elektrode 2 verwendet ist.

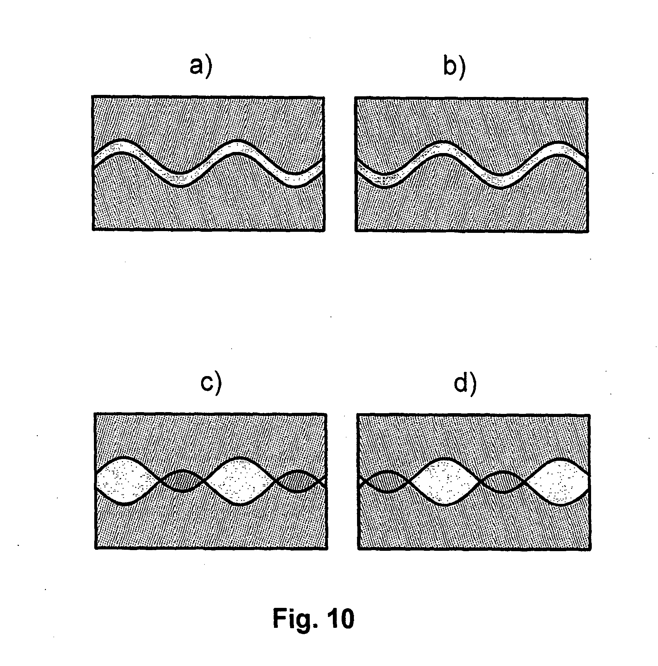

Die Metallisierungsflächen der Keramikröhrchen 18 sind in abgewickelter Form in Fig. 10a bis 10d gezeigt, wobei Fig. 10a und 10c die Metallisierung der Außenfläche und Fig. 10b und 10d die Metallisierung der Innenfläche darstellen. Zur Veranschaulichung der Kontaktierung beider Seiten sind die Innen- und Außenflächen zusammen dargestellt, wobei jeweils nur ein elektrischer Pol des Bauteils gezeigt ist. An den Stellen, an denen sich die Innen- und Außenfläche der Metallisierung eines Pols überlappen, sind Durchkontaktierungen zum Verbinden der Innen- und Außenfläche hergestellt.

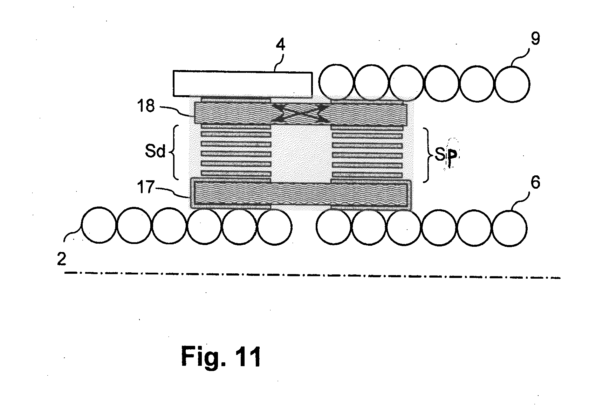

Fig. 11 zeigt eine weitere Ausführungsform der Stimulationselektrodenleitung 5, in der zwei parallel leitfähige Wendeln Sp, Sd zwischen zwei Keramikröhrchen angeordnet sind.

Das Keramikröhrchen 18, das mit der zweiten Elektrode 4 und der Außenzuleitung 9 in Verbindung steht, besitzt eine integrierte Leitungsüberkreuzung, wie in Fig. 10 dargestellt. Das Keramikröhrchen 17, das mit der ersten Elektrode 2 und der Innenzuleitung 6 in Verbindung steht, besitzt hingegen nur auf der Außenfläche Metallisierungen. Die proximale und distale Spirale Sp, Sd gewährleisten die elektrische Verbindung, zwischen der Innenzuleitung 6 und der zweiten Elektrode 4 bzw. zwischen der Außenzuleitung 9 und der ersten Elektrode 2, durch die Leitungsüberkreuzung des Keramikröhrchens 18.

Die Ausführung der Erfindung ist nicht auf die oben beschriebenen Beispiele und hervorgehobene Aspekte beschränkt, sondern ebenso in einer Vielzahl von Abwandlungen möglich, die im Rahmen fachgemäßen Handelns liegen.

## Patentansprüche

1. Implantierbare Stimulationselektrodenleitung (5) für medizinische Anwendungen, umfassend:
eine koaxiale Leitungsstruktur (10) mit einer Innenzuleitung (6) und einer von der Innenzuleitung (6) elektrisch isolierten Außenzuleitung (9) und eine erste und eine zweite Elektrode (2, 4), die am oder nahe dem distalen Ende der Leitungsstruktur (10) vorgesehen sind,
wobei die erste Elektrode (2) an der Spitze der Leitungsstruktur (10) und die zweite Elektrode (4) proximal von der ersten Elektrode (2) angeordnet ist,
wobei die Innenzuleitung (6) zur zweiten Elektrode (4) und die Außenzuleitung (9) zur ersten Elektrode (2) geführt ist,
**dadurch gekennzeichnet, dass** die implantierbare Stimulationselektrodenleitung Mittel zur aktiven Fixierung und ein zwischen der Innenzuleitung (6) und der ersten Elektrode (2) angeordnetes, aus isolierendem Material bestehendes, Übertragungselement (13) zur Kraftübertragung von der Innenzuleitung (6) auf die erste Elektrode (2) umfasst.

2. Stimulationselektrodenleitung (5) nach Anspruch 1, wobei die Innenzuleitung (6) relativ zu der Außenzuleitung (9) drehbar ist und die erste Elektrode (2) gegenüber der zweiten Elektrode (4) durch die Drehung der Innenzuleitung (6) verschiebbar ist.

3. Stimulationselektrodenleitung (5) nach Anspruch 1 oder 2, wobei die Innenzuleitung (6) relativ zur Außenzuleitung (9) durch Schrauben verschiebbar ist.

4. Stimulationselektrodenleitung (5) nach einem der Ansprüche 1 bis 3, weiter umfassend flexible elektrische Verbindungsleitungen (14, 15) zur elektrischen Verbindung zwischen der Innenzuleitung (6) und der zweiten Elektrode (4) und zwischen der Außenzuleitung (9) und der ersten Elektrode (2).

5. Stimulationselektrodenleitung (5) nach Anspruch 4, wobei die elektrischen Verbindungsleitungen (14, 15) aus dehnbarem Material bestehen.

6. Stimulationselektrodenleitung (5) nach Anspruch 4 oder 5, wobei die elektrischen Verbindungsleitungen (14, 15) eine verlängerbare Wendelstruktur aufweisen.

7. Stimulationselektrodenleitung (5) nach Anspruch 6, wobei die Wendelstruktur um die Innenzuleitung (6) herum ausgebildet ist.

8. Stimulationselektrodenleitung (5) nach einem der Ansprüche 1 bis 3, weiter umfassend eine Spiralfeder (16) mit einem inneren und einem äußeren Teil (161, 162) zur elektrischen Verbindung der Innenzuleitung (6) mit der zweiten Elektrode (4) und der Außenzuleitung (9) mit der ersten Elektrode (2), wobei der innere Teil (161) gegenüber dem äußeren Teil (162) verdrehbar ist.

9. Stimulationselektrodenleitung (5) nach Anspruch 8, wobei die Spiralfeder (16) ein gewickeltes Substrat (S) aufweist, auf dem sich beidseitig eine Metallisierung (M) befindet, wobei die Metallisierung (M) auf einer Seite des Substrats (S) die Verbindung der Innenzuleitung (6) mit der zweiten Elektrode (4) und die Metallisierung (M) auf der anderen Seite des Substrats (S) die Verbindung der Außenzuleitung (9) mit der ersten Elektrode (2) herstellt.

10. Stimulationselektrodenleitung (5) nach Anspruch 8 oder 9, weiter umfassend einen zwischen der Spiralfeder (16) und der ersten bzw. zweiten Elektrode (2, 4) und/oder der Innen- bzw. Außenzuleitung (6, 9) angeordneten leitfähigen Adapter (17) zur Verbesserung der mechanischen Stabilität der Verbindung zwischen der Spiralfeder (16) und den Zuleitungen (6, 9) bzw. den Elektroden (2, 4).

11. Stimulationselektrodenleitung (5) nach einem der Ansprüche 1 bis 3, weiter umfassend eine röhrenförmige Struktur (18) mit integrierter Leitungsüberkreuzung zur elektrischen Verbindung der Innenzuleitung (6) mit der zweiten Elektrode (4) und der Außenzuleitung (9) mit der ersten Elektrode (2).

12. Stimulationselektrodenleitung (5) nach Anspruch 11, wobei die röhrenförmige Struktur (18) mit einer distalen leitfähigen Wendel (Sd) zur Verbindung der Außenzuleitung (9) mit der ersten Elektrode (2) und mit einer proximalen leitfähigen Wendel (Sp) zur Verbindung der Innenzuleitung (6) mit der zweiten Elektrode (4) gekoppelt ist.

13. Stimulationselektrodenleitung (5) nach Anspruch 1, wobei das Übertragungselement (13) beim Drehen der Innenzuleitung (6) verlängerbar ist.

14. Implantierbare Stimulationsanordnung insbesondere Herzschrittmacher (3) oder Defibrillator oder Neurostimulator, umfassend eine Stimulationselektrodenleitung (5) nach einem der Ansprüche 1 bis 13.

## Claims

1. An implantable stimulation electrode lead (5) for medical applications, comprising:
a coaxial lead structure (10) having an inner lead (6) and an outer lead (9), which is electrically insulated with respect to the inner lead (6), and a first and a second electrode (2, 4), which are provided at or near the distal end of the lead structure (10),
wherein the first electrode (2) is arranged at the tip of the lead structure (10) and the second electrode (4) is arranged proximally with respect to the first electrode (2),
wherein the inner lead (6) is routed to the second electrode (4) and the outer lead (9) is routed to the first electrode (2),
**characterized in that** the implantable stimulation electrode lead comprises means for active fixation, and comprises a transmission element (13), which is arranged between the inner lead (6) and the first electrode (2) and consists of insulating material, for transmitting force from the inner lead (6) to the first electrode (2).

2. The stimulation electrode lead (5) according to claim 1, wherein the inner lead (6) is rotatable relative to the outer lead (9), and the first electrode (2) is displaceable relative to the second electrode (4) by means of the rotation of the inner lead (6).

3. The stimulation electrode lead (5) according to claim 1 or 2, wherein the inner lead (6) is displaceable relative to the outer lead (9) by means of screwing.

4. The stimulation electrode lead (5) according to any one of claims 1 to 3, further comprising flexible electrical connecting leads (14, 15) for the electrical connection between the inner lead (6) and the second electrode (4), and between the outer lead (9) and the first electrode (2).

5. The stimulation electrode lead (5) according to claim 4, wherein the electrical connecting leads (14, 15) consist of stretchable material.

6. The stimulation electrode lead (5) according to claim 4 or 5, wherein the electrical connecting leads (14, 15) consist of an extensible helical structure.

7. The stimulation electrode lead (5) according to claim 6, wherein the helical structure is formed around the inner lead (6).

8. The stimulation electrode lead (5) according to any one of claims 1 to 3, further comprising a spiral spring (16) having an inner and an outer part (161, 162) for electrically connecting the inner lead (6) to the second electrode (4) and for electrically connecting the outer lead (9) to the first electrode (2), wherein the inner part (161) is rotatable relative to the outer part (162).

9. The stimulation electrode lead (5) according to claim 8, wherein the spiral spring (16) has a wound substrate (S), both sides of which have a metallization (M), wherein the metallization (M) on one side of the substrate (S) establishes the connection of the inner lead (6) to the second electrode (4) and the metallization (M) on the other side of the substrate (S) establishes the connection of the outer lead (9) to the first electrode (2).

10. The stimulation electrode lead (5) according to claim 8 or 9, further comprising a conductible adapter (17), which is arranged between the spiral spring (16) and the first and second electrode (2, 4), respectively, and/or the inner and outer lead (6, 9), respectively, for improving the mechanical stability of the connection between the spiral spring (16) and the leads (6, 9) and the electrodes (2, 4), respectively.

11. The stimulation electrode lead (5) according to any one of claims 1 to 3, further comprising a tubular structure (18) having an integrated lead crossover for electrically connecting the inner lead (6) to the second electrode (4) and for electrically connecting the outer lead (9) to the first electrode (2).

12. The stimulation electrode lead (5) according to claim 11, wherein the tubular structure (18) is coupled to a distal conductive helix (Sd) for connecting the outer lead (9) to the first electrode (2) and is coupled to a proximal conductive helix (Sp) for connecting the inner lead (6) to the second electrode (4).

13. The stimulation electrode lead (5) according to claim 1, wherein the transmission element (13) can be extended when the inner lead (6) is rotated.

14. An implantable stimulation arrangement, in particular a cardiac pacemaker (3) or defibrillator or neurostimulator, comprising a stimulation electrode lead (5) according to any one of claims 1 to 13.

## Revendications

1. Ligne d'électrode de stimulation (5) implantable pour des utilisations dans le domaine médical, comprenant :
une structure de ligne coaxiale (10) avec une ligne d'alimentation interne (6) et une ligne d'alimentation externe (9), isolée électriquement de la ligne d'alimentation interne (6), et, une première et une deuxième électrodes (2, 4) qui sont prévues à l'extrémité distale, ou près de l'extrémité distale de la structure de ligne (10),
dans laquelle la première électrode (2) est disposée à la pointe de la structure de ligne (10) et la deuxième électrode (4) est proximale par rapport à la première électrode (2),
dans laquelle la ligne d'alimentation interne (6) est menée vers la deuxième électrode (4) et la ligne d'alimentation externe (9) est menée vers la première électrode (2),
**caractérisée en ce que** la ligne d'électrode de stimulation implantable comprend des moyens pour une fixation active et un élément de transmission (13), disposé entre la ligne d'alimentation interne (6) et la première électrode (2), constitué d'un matériau isolant, destiné à la transmission d'une puissance de la ligne d'alimentation interne (6) vers la première électrode (2).

2. Ligne d'électrode de stimulation (5) selon la revendication 1, dans laquelle la ligne d'alimentation interne (6) peut être en rotation par rapport à la ligne d'alimentation externe (9) et la première électrode (2) peut être déplacée par rapport à la deuxième électrode (4) par la rotation de la ligne d'alimentation interne (6).

3. Ligne d'électrode de stimulation (5) selon les revendications 1 ou 2, dans laquelle la ligne d'alimentation interne (6) peut être déplacée par rapport à la ligne d'alimentation externe (9) par un vissage.

4. Ligne d'électrode de stimulation (5) selon l'une des revendications 1 à 3, comprenant en outre des lignes de liaison (14, 15) électriques souples pour une liaison électrique entre la ligne d'alimentation interne (6) et la deuxième électrode (4) et entre la ligne d'alimentation externe (9) et la première électrode (2).

5. Ligne d'électrode de stimulation (5) selon la revendication 4, dans laquelle les lignes de liaison (14, 15) électriques sont constituées dans un matériau extensible.

6. Ligne d'électrode de stimulation (5) selon les revendications 4 ou 5, dans laquelle les lignes de liaison (14, 15) électriques présentent une structure en hélice étirable.

7. Ligne d'électrode de stimulation (5) selon la revendication 6, dans laquelle la structure en hélice est construite autour de la ligne d'alimentation interne (6).

8. Ligne d'électrode de stimulation (5) selon l'une des revendications 1 à 3, comprenant en outre un ressort en spirale (16) avec une partie interne et une partie externe (161, 162) pour une liaison électrique de la ligne d'alimentation interne (6) avec la deuxième électrode (4) et celle de la ligne d'alimentation externe (9) avec la première électrode (2), dans laquelle la partie interne (161) peut être en rotation par rapport à la partie externe (162).

9. Ligne d'électrode de stimulation (5) selon la revendication 8, dans laquelle le ressort en spirale (16) présente un substrat (S) bobiné sur lequel se trouve une métallisation (M) sur les deux faces, dans laquelle la métallisation (M) sur une face du substrat (S) réalise la liaison de la ligne d'alimentation interne (6) avec la deuxième électrode (4) et la métallisation (M) sur l'autre face du substrat (S) réalise la liaison de la ligne d'alimentation externe (9) avec la première électrode (2).

10. Ligne d'électrode de stimulation (5) selon les revendications 8 ou 9, comprenant en outre un adaptateur (17) conducteur disposé entre le ressort en spirale (16) et la première, respectivement, la deuxième, électrode (2, 4) et/ou la ligne d'alimentation interne, respectivement, externe (6, 9) pour une amélioration de la stabilité mécanique de la liaison entre le ressort en spirale (16) et les lignes d'alimentations (6, 9), respectivement, les électrodes (2, 4).

11. Ligne d'électrode de stimulation (5) selon l'une des revendications 1 à 3, comprenant en outre une structure en forme de tuyau (18) avec un croisement de lignes intégré pour la liaison électrique de la ligne d'alimentation interne (6) avec la deuxième électrode (4) et de la ligne d'alimentation externe (9) avec la première électrode (2).

12. Ligne d'électrode de stimulation (5) selon la revendication 11, dans laquelle la structure en forme de tuyau (18) est couplée avec une spirale (Sd) conductrice distale pour la liaison de la ligne d'alimentation externe (9) avec la première électrode (2) et avec une spirale (Sp) conductrice proximale pour la liaison de la ligne d'alimentation interne (6) avec la deuxième électrode (4).

13. Ligne d'électrode de stimulation (5) selon la revendication 1, dans laquelle l'élément de transmission (13) peut être allongé par une rotation de la ligne d'alimentation interne (6).

14. Ensemble de stimulation implantable, notamment stimulateur cardiaque (3) ou défibrillateur, ou neurostimulateur, comprenant une ligne d'électrode de stimulation (5) selon l'une des revendications 1 à 13.
